# EUROPEAN PATENT APPLICATION

(11) **EP 2 208 532 A1**
(43) Date of publication of application: **21.07.2010**
(21) Application number: 09160987.5
(22) Date of filing: 25.05.2009
(51) Int. Cl.: B01L 3/00, G01N 33/543, G01N 35/00, G01N 1/40

(54) **Distribution of particles in chamber by application of magnetic field**

(30) Priority: 30.12.2008 EP 08173113
(71) Applicant: Atonomics A/S, 2450 Copenhagen SV (DK)
(72) Inventor: Warthoe, Peter, 2100, Copenhagen Ø (DK); Mentzel, Søren, 2300, Copenhagen S (DK); Mikkelsen, Jens, 2750, Ballerup (DK); Sorensen, Iben Schildt, 2200 Copenhagen N (DK)
(74) Representative: Kitchen, Steven Richard

(57) **Abstract**

The present invention relates to a device and a method for clustering and subsequently distributing a plurality of paramagnetic particles (7) in a small volume liquid in a chamber, said chamber having a first wall (12) and an opposite second wall (13), said method comprising the steps of a) providing a liquid comprising the plurality of paramagnetic particles, b) subjecting the paramagnetic particles to a first magnetic field by means of a first field generating means (25) having first and second mutually spaced opposite poles and defining a first pole axis (26) extending between the poles, the field generating means being arranged relative to the first wall so that the first pole axis (a₁) forms an angle (v₁) of less than 60° with the first wall, c) moving the magnetic field in a first moving direction having a movement component in the direction of the first pole axis, or, alternatively, changing the polarity of the magnetic field one or more times, and, d) removing the magnetic field.

## Description

### Technical Field

The present invention relates to a method and a device for clustering and subsequently homogenously distributing a plurality of paramagnetic particles in a small volume liquid.

### Background

The use of microfluidic systems of reduced dimension and containing magnetic or paramagnetic particles for analytical applications are well-known and magnetic or paramagnetic nano- and microparticles have become a hot topic in several technical fields of research. Functional nano- and microparticles ('beads') offer a large specific surface for chemical binding and a polymer colloid or microsphere solution has a low viscosity compared to solutions having the same amount of solid, giving it special properties. Thus, such small particles can be advantageously used as a 'mobile substrate' for bio-assays. One of the most prominent advantages of magnetic beads however, lies in the fact that the particles can be magnetically probed and manipulated using permanent magnets or electromagnets, independent of normal chemical or biological processes.

Some of the classical applications, like magnetic separation, have already found their way down to miniaturized fluidic or 'lab-on-a-chip' systems that strongly limit the consumption of samples and reagents; in such systems, magnetic beads effectively provides a chemically active substrate with a large surface-to-volume ratio. The booming area of miniaturized applications of magnetic beads offers many exciting possibilities for future developments. It is a highly multidisciplinary area, requiring contributions from inorganic chemistry involved in the preparation of the magnetic beads, through biochemistry and medical science to allow for their functionalization and, of course, the basic physics of magnetism and magnetic materials. Nearly all important functions in a bio-assay can be realized using magnetic beads: sample purification, providing a solid substrate to the sample, mixing, labelling, manipulation and transport and, finally, separation.

Magnetic or paramagnetic beads coated with labels or capture molecules are widely used for the detection of biomarkers and biological molecules, and magnetic particles based assay has become a standard in modern chemical and biological diagnostics. Detection of biological molecules is usually accomplished using biomolecular recognition between the target molecule and a specific receptor (e.g. an antibody) that is tagged with a label. The label may for example be a radioisotope, enzyme, fluorescent molecule or a charged molecule or any combination thereof. Recently, also magnetic beads have been used as labels for biosensing.

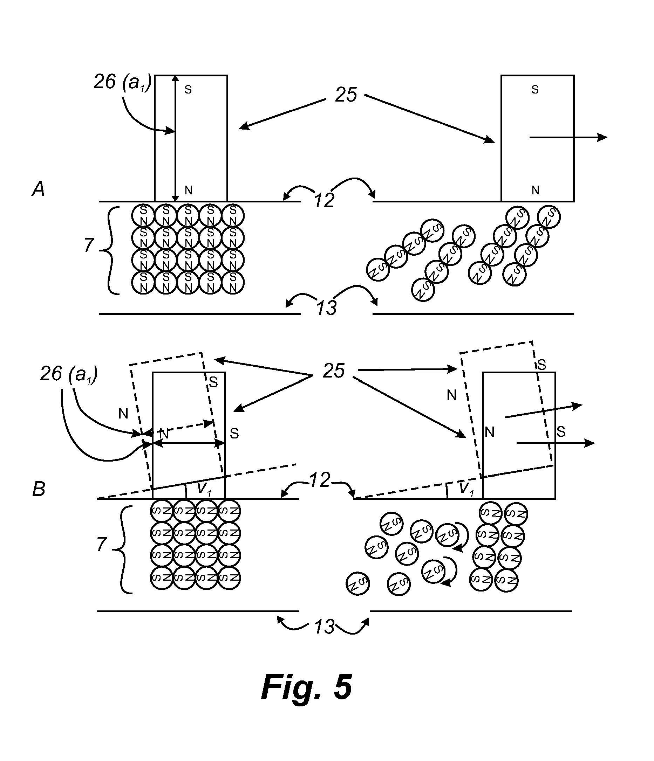

These magnetic and paramagnetic beads are manipulated by use of means generating a magnetic field. The positioning of the magnetic field (in terms of polarity) relative to the liquid comprising the paramagnetic material is traditionally such that the poles of the magnetic field are placed directly perpendicular to the liquid containing the particles. In other words, magnetic devices known in the art generate a magnetic field having first and second opposite poles arranged so that the paramagnetic particles are subjected to a magnetic field by means of field-generating means having first and second mutually spaced opposite poles, which define a pole axis extending between the poles, the field generating means being arranged relative to the liquid so that the pole axis forms an angle of 90° with the liquid. An example of a prior art device is shown in Fig. 5A.

However, when small volume solutions containing paramagnetic or magnetic beads are subject to magnetic manipulation using the conventional manipulation techniques, an unsatisfactory detection limit and a significant standard deviation between comparative assays have surprisingly been observed. This has resulted in an unsatisfactorily high CV-value (detection mean/standard deviation) with the result that small volume detector devices using paramagnetic bead technology to date have not been widely approved as reliable detection instruments.

The present inventors aimed at finding the cause of the observed unsatisfactorily high CV-value (detection mean/standard deviation). During these investigations, it was observed that the manipulation of magnetic particles in a fluidic environment according to the prior art techniques resulted in several physical constraints. The particles which are continuously exposed to the magnetic fields were observed to stick together and agglomerate, thereby losing several of their main advantages: e.g. the active/coated surface of the particle that is in contact with surrounding bio-chemical liquid is drastically reduced which seriously compromises the assay performance. Further, analyte species captured in between particle agglomerates may not respond properly to the assay detection means (particles shield the signal from other particles), which further reduces the signal-to-noise ratio. In certain applications where magnetic or paramagnetic particles, which are not bound to a target analyte, are to be discarded prior to the application of the detection means, several unbound magnetic or paramagnetic particles are trapped in agglomerates and cannot be removed. These undesired effects drastically increase background signal and reduce analyte based signal. Consequently, an unsatisfactory reproducibility and reliability is observed.

Accordingly, there is a need in the art for improved methods and devices which can increase sensitivity and reduce standard deviations when detecting analyte presence and concentrations in small volume samples.

Thus, one object of the invention is to provide improved methods and devices which can increase sensitivity and reduce standard deviations when detecting analyte presence and concentrations in small volume samples.

### Brief disclosure of the invention

Surprisingly, the problem of the invention was solved by simply changing the direction of the magnetic fields applied to the sample from a situation wherein the poles are perpendicular to the liquid containing the particles to a situation where the poles are substantially parallel with respect to the liquid containing the particles. The positioning of the poles of the magnetic field in relation the paramagnetic particles has until present not even been considered to be of any importance.

Accordingly, the invention relates to a method for clustering and subsequently distributing a plurality of paramagnetic particles in a small volume liquid in a chamber, said chamber having a first wall and an opposite second wall, said method comprising the following steps;
a) providing a liquid comprising the plurality of paramagnetic particles;
b) subjecting the paramagnetic particles to a first magnetic field by means of a first field generating means having first and second mutually spaced opposite poles and defining a first pole axis extending between the poles, the field generating means being arranged relative to the first wall so that the first pole axis (a₁) forms an angle (v₁) of less than 89°, and preferably less than 60°, with the first wall;
c) moving the magnetic field in a first moving direction having a movement component in the direction of the first pole axis, or, alternatively, changing the polarity of the magnetic field one or more times, and
d) removing the magnetic field.

Further, the invention relates to a system for clustering and subsequently distributing a plurality of paramagnetic particles in a chamber, said system comprising a chamber device and a magnetic device, said chamber device comprising a first wall and an opposite second wall defining a chamber arranged for receiving and containing the plurality of paramagnetic particles, and said magnetic device comprising a) receiving means for receiving said chamber device and comprising a first magnetic field generating means for generating a first magnetic field having first and second mutually spaced opposite poles, and defining a first pole axis (a₁) extending between the poles, the first field generating means being arranged relative to the first wall of a the chamber device, received in the receiving means so that paramagnetic particles in the chamber are subjected to the field and so that the first pole axis (a₁) forms an angle (v₁) of less than 60° with the first wall of the chamber device received in the receiving means, and further comprising means for changing the magnetic field.

Further, the invention relates to a method according to claim 15 for the detection of the presence or absence of a target analyte in a sample consisting of less than 200 µl liquid.

### Brief Description of the Drawings

The invention is explained in detail below with reference to the drawings, in which
Fig. 1 discloses a perspective view of an embodiment of a chamber device of a system according to the invention, a cover being removed for illustrative purposes.
Fig. 2 is a schematic presentation of a chamber device as shown in Fig. 1.
Fig. 3 is an enlarged schematic sectional view of a chamber of the chamber device.
Fig. 4 is a schematic view of an embodiment of a system according to the invention
Fig. 5 is a schematically illustration of the main principles of the invention compared to the main principles of the prior art.

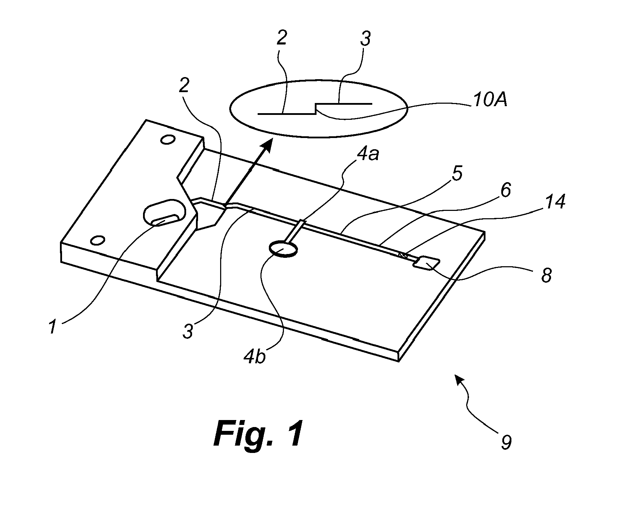

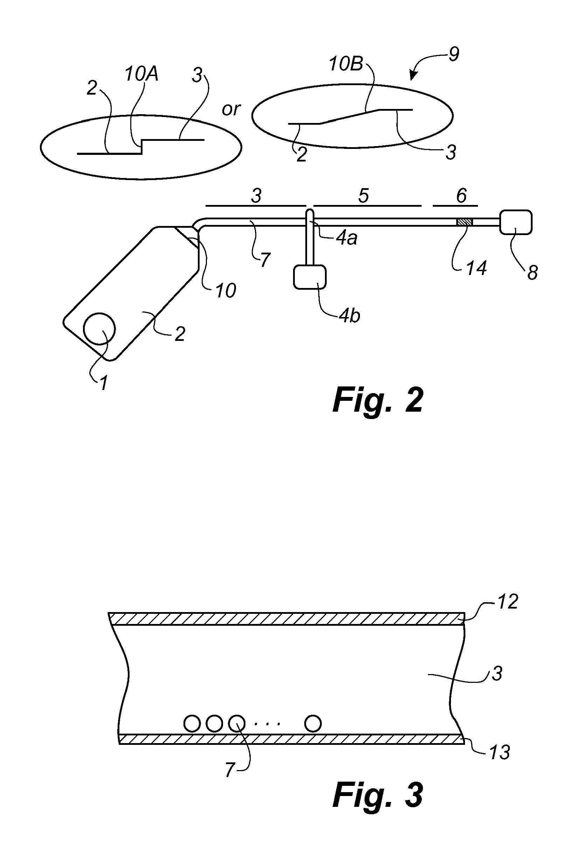

The chamber device (9) shown in Fig. 1 and Fig. 2 comprises a microfluid channel having a first part (3) and a second part (5, 6). The first part (3) is a chamber formed as a capillary channel and is the chamber to be subjected to the magnetic field when carrying out the method according to the invention. However, also the second part (5, 6) may be subjected to the magnetic field. The paramagnetic particles may be present in the first part prior to introduction of the liquid or may be introduced with the liquid.

The chamber device further comprises an application zone (1) for applying a liquid comprising the analyte (e.g. blood) to the capillary channel and a separation chamber (2) wherein the liquid is separated into a retentate and a liquid comprising the analyte. The separation chamber (2) is connected to a capillary channel (3), in this particular embodiment via a filter and a physical barrier (10), which can be formed as a step (10a) or a ramp/slope (10b).

A collection chamber (4a) is formed between the capillary channel (3) and the second part of the microfluid channel. The second part comprises a washing chamber (5) in which the paramagnetic particles are washed after binding with the analyte in the chamber (3), and a detection chamber (6) in which detection takes place through a detection window (14). The inventive method may also be used in these parts of the microfluid system to avoid agglomeration of the particles. Washing and detection solution can be supplied to the second part (5, 6) of the microfluid channel. A waste outlet (4b) is arranged between the capillary channel (3) and the second part (5, 6) of the microfluid channel.

As seen in Fig. 3, the capillary channel (3) is defined by a first wall (12) and an opposite second wall (13) and the capillary channel contains a plurality of paramagnetic particles (7).

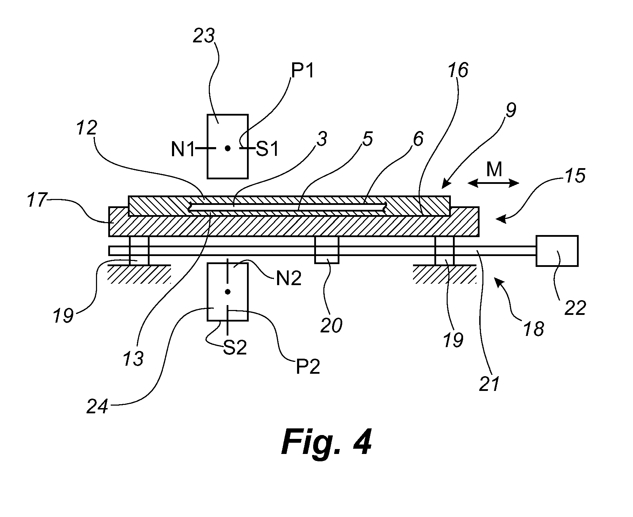

Referring to Fig. 4, the system according to the invention for clustering and subsequently distributing a plurality of paramagnetic particles comprises the chamber device (9) described previously and a magnetic device (15).

The magnetic device (15) comprises receiving means for receiving the chamber device (9) and being formed by a recess (16) in a slide (17), the recess (16) having a contour essentially corresponding to the chamber device (9). The chamber device (9) is placed in the recess with the second wall (13) facing the bottom of the recess (16).

As seen in Fig. 4, the slide (17) is horizontally movable parallel with the longitudinal direction of the microfluid channel (3, 5, 6) of the chamber device by means of a spindle/nut arrangement (18). The spindle/nut arrangement comprises two mutually spaced supports (19), supporting and guiding the slide longitudinally by means of not shown guides and a nut (20) connected to the slide (17). A spindle (21) extends through bearings (19) in the supports (19) and through the nut (20). The spindle (21) can be driven to rotation by means of a motor (22) for moving the slide (17) horizontally in a first direction and an opposite direction as shown by means of the arrow (M).

As will be explained later, the spindle/nut arrangement provides means for changing the first magnetic field.

The magnetic device further comprises a first magnetic field generating means which in the embodiment shown is formed by a first permanent magnet (23) having first and second spaced opposite poles N1 and S1 defining a first pole axis (P₁).

The magnet (23) has a fixed position relative to the slide (17) and is arranged such in relation to the chamber device (9) received in the recess (16) of the slide (17) that at least the paramagnetic particles (7) contained in the capillary channel (3) is subjected to the magnetic field of the first magnet (23), when applied. In the embodiment shown the first pole axis is essentially parallel to the longitudinal direction of the microfluid channel of the chamber device (9). It is not essential that the pole axis or the movement of the field is parallel to the capillary channel for the function of the device. The magnetic field must however be able to move the paramagnetic particles contained in the capillary channel in the longitudinal direction of the microfluid channel of the chamber device (9).

The means for changing the magnetic field may also be used as means for removing the magnetic field, as a rapid movement of the magnetic field will not be followed by the particles, which will then drop off the first wall due to gravity. Alternatively, removal of the magnetic field may be done by switching off the power in case of an electromagnet.

As shown in Fig. 4, the magnetic device (15) additionally comprises a second magnet field generating means which in the embodiment shown is formed by a second permanent magnet (24) having first and second spaced opposite poles N2 and S2 and defining a second pole axis P2 extending between the poles.

In the embodiment shown, the second magnet (24) is arranged such that the second pole axis is perpendicular to the longitudinal direction of the microfluid channel of the chamber device and so that at least the paramagnatic particles (7) contained in the capillary channel is subjected to the magnetic field of the second magnet when applied.

Preferably, only one of the magnets (23, 24) is functioning at one time. In an alternative embodiment, when the first magnet (23) is applied, the magnetic drag force is preferably stronger than the second magnet (24). The magnet (24) may also be arranged as magnet (23), i.e. with the poles directed according to the invention.

Referring to Fig. 5, this figure illustrates the principle of the invention (B) with the pole axis (26) of the magnetic field substantially parallel to the first wall of the chamber device compared to a prior art situation where the pole axis of the magnetic field are substantially perpendicular to the first wall of the chamber device (A).

The magnetic field generating means (25) have first (N) and second (S) mutually spaced opposite poles defining a first pole axis (26) extending between the poles, the field generating means being arranged relative to the first wall so that the first pole axis (a₁) forms an angle (v₁) of less than 60° with the first wall (12). (13) illustrates the opposite second wall (13) and the (7) the plurality of paramagnetic particles.

When the magnetic field is applied to at least a portion of said channel by way of the magnetic field generating means paramagnetic particles are attracted to the first inner surface of the chamber, thereby forming clusters of particles. When moving the magnetic field generating means longitudinally (indicated by the arrows) in relation to the chamber, the clusters of particles in (A) will not shift magnetic polarity (indicated by the half circular arrows) and be released as rows/clusters of particles. Without wishing to be bound by theory, it is believed that in (B), the particles will shift magnetic polarity and be released as smaller clusters and/or single particles and by gravity distributed homogeneously on the second wall (13). At least practice has proved that in B), the particles are released as small clusters and/or single particles, as appears from the results obtained in example 1.

### Definitions

In the context of the present invention, by "paramagnetic particles" is meant particles which are magnetic in the presence of an externally applied magnetic field, but are not magnetic prior to being subjected to a magnetic field. The paramagnetic particles according to the invention are capable of binding and immobilising a particular analyte species of interest, e.g. by being coated with a receptor or antibody.

In the context of the present invention, by "homogeneously distributing" is meant a distribution where most of the particles in a cluster of the particles primarily (preferable) is released as single particles and secondarily in smaller clusters separated substantially homogenously over a given area of a capillary channel.

In the context of the present invention, by "capillary channel" is meant a narrow tube or channel through which a fluid can pass. Preferably the diameter (or width) of a capillary channel according to the invention is less than 10 mm. Even more preferred the diameter of a capillary channel according to the invention is less than 5mm, such as less than 4 mm, or less than 3 mm or even less than 2 mm. In a most preferred embodiment the capillary channel has a diameter of 1 mm or less, e.g. 0,2-1.0 mm. The channels may also be formed of non-circular shapes, e.g. rectangular or triangular, in which case the "diameter" refers to the mean distance from the center of the cannel to the periphery.

### Disclosure of the Invention

An object of the present invention is to develop a system and a method for quantitatively detecting the presence or absence of a target analyte in a small liquid sample, wherein the accuracy and precision of a given assay is improved, especially for small quantities of analyte, and wherein the background unspecific signal is reduced or eliminated, and wherein the signal arising from the analyte is increased.

Surprisingly, it was found that positioning the poles of the magnetic field relative to the movement direction of the paramagnetic material was of great importance. Accordingly, it was found that arranging the magnetic field such that the poles of the magnetic field having first and second mutually spaced opposite poles and defining a first pole axis extending between the poles such that the field generating means are arranged relative to the first wall of the chamber comprising the paramagnetic particles so that the first pole axis (a₁) forms an angle (v₁) of less than 60° with the first wall of the container provided a significant improvement in terms of signal intensity and reduction of background. Further it was found that the inventive arrangement of the magnetic field resulted in significantly higher reproducibility of the assay.

The findings of the present inventors are particularly surprising as it is generally believed that paramagnetic material do not retain any substantial magnetization in the absence of an externally applied magnetic field because thermal motion causes the spins to become randomly oriented without it. Thus the total magnetization will in theory drop to zero when an applied field is removed. Further, paramagnetic materials follow the Curie type law but with exceptionally large values for the Curie constants. Thus, it is generally believed that the paramagnetic particles are not magnetic and do not cluster in the absence of a magnetic field.

However, and without wishing to be bound by theory, it is believed that the cause of the problem which was solved according to the present invention is that when placed in a magnetic field, paramagnetic particles do in fact acquire a magnetic moment that is sufficient to initiate an interaction between the individual particles by magnetic dipole interaction, and that this dipole interaction is not removed simply by removing the magnetic field. This interaction may induce a spontaneous clustering of the particles into larger, often complicated, structures, and agglomerates, which again causes a decreased signal and reproducibility of the assay.

By combining microfluid and magnetic particle technology in a special constellation, the present inventors found that it was possible to fulfil the critical parameters and at the same time apply the constellation in a relative small handheld instrument (below 500 gram), capable of analysing samples of less than 200µl.

Accordingly, the invention relates in one embodiment to a method for clustering and subsequently distributing a plurality of paramagnetic particles in a small volume liquid in a chamber, said chamber having a first wall and an opposite second wall, said method comprising the following steps;
a) providing a liquid comprising the plurality of paramagnetic particles;
b) subjecting the paramagnetic particles to a first magnetic field by means of a first field generating means having first and second mutually spaced opposite poles and defining a first pole axis extending between the poles, the field generating means being arranged relative to the first wall so that the first pole axis (a₁) forms an angle (v₁) of less than 60° with the first wall;
c) moving the magnetic field in a first moving direction having a movement component in the direction of the first pole axis, or, alternatively, changing the polarity of the magnetic field one or more times, and
d) removing the magnetic field.

When the paramagnetic material has been in contact with a liquid containing a particular analyte, and when that analyte has been immobilized on the surface of the paramagnetic particles, e.g. by use of receptors or antibodies coated on the surface of the paramagnetic particles, one or more additional manipulation steps are often required. Thus, manipulation of paramagnetic particles in small volume samples often requires several steps of treatment, such as one or more steps of sample application and contact between particles and sample containing the particular analyte, one ore more washing steps, and one ore more steps of application of other liquids used for detection purposes. During each of these liquid treatment steps it is highly preferred that the particles do not agglomerate, thereby possibly resulting in a loss of intensity and reproducibility of the assay.

Accordingly, in one preferred embodiment according to the invention, the steps a) to d) are repeated one or more times. Preferably, the introduction of subsequent liquids results in repeating the steps of the invention.

It was further observed that movement of the magnetic field back and forth one or more times in the direction of the pole axis resulted in an improved result. Accordingly, in one preferred embodiment according to the invention, the method comprises subsequent to step c) and before step d), a step c₁) of moving the magnetic field in a reverse moving direction having a movement component in the reverse direction of the first pole axis (a₁). Preferably, the steps c) and c₁) are repeated one or more times. Alternatively, changing the polarity of the magnetic field one or more times may also provide the required effect.

The placement of the first magnetic field according to the invention is an essential embodiment of the invention in that the angle (v₁) between the pole axis and the liquid cannot be 90°. However, in theory, angles of less than 89° would be sufficient to obtain at least a reduction of the agglomeration of the paramagnetic particles. However, in practice, angles of less than 60° are preferable for obtaining an effect which is substantially superior to prior art techniques. Thus, in one embodiment the invention relates to a method as described above wherein the first field generating means are arranged relatively to the first wall so that the angle (v₁) between the first pole axis and the first wall is between 0° and 60°.

In one embodiment, the first field generating means are arranged relative to the first wall so that the angle (v₁) between the first pole axis and the first wall is between 0° and 50°.

In one embodiment, the first field generating means are arranged relative to the first wall so that the angle (v₁) between the first pole axis and the first wall is between 0° and 40°.

In one embodiment, the first field generating means are arranged relative to the first wall so that the angle (v₁) between the first pole axis and the first wall is between 0° and 20°.

In one embodiment, the first field generating means are arranged relative to the first wall so that the angle (v₁) between the first pole axis and the first wall is between 0° and 10°.

In one embodiment, the first field generating means are arranged relative to the first wall so that the angle (v₁) between the first pole axis and the first wall is 0°.

The magnetic field generating means may be a permanent magnet or an electromagnet. The magnetic field generating means may be a rotating permanent magnet, or an electromagnet where the poles can be switched during the application and moving of the magnetic field.

The magnetic field must be moved so as to subject the magnetic particles to movement along the first wall of the device. Alternatively, changing the polarity of the magnetic field one or more times may initiate rotation of the particles. It is not essential that the pole axis or the movement of the field is parallel to the chamber for the function of the device. The magnetic field must however be able to move the paramagnetic particles contained in the capillary channel in the longitudinal direction of the microfluid channel of the chamber device. Thus, in a preferred embodiment of the method according to the invention, the first moving direction is essentially in the direction of the first pole axis a₁). Further, it is preferred that the movement is reversed. Thus, in a preferred embodiment of the method according to the invention, the movements are reversed in a movement direction that is essentially in the reverse direction of the first pole axis a₁).

In on particular embodiment of the invention, the chamber is formed as the chamber described in e.g. WO06134546A and comprises a space containing a plurality of paramagnetic particles, and a magnetic field generating means for generating a magnetic field and an opposite magnetic field generating means for generating an opposite magnetic field.

In this particular embodiment, the invention relates to a method according to the above comprising subsequent to step d), a step e) of subjecting the paramagnetic particles to a second magnetic field by means of a second field generating means having first and second mutually spaced opposite poles and defining a second pole axis a₂) extending between the poles, the second field generating means being arranged relative to the second wall so as to attract the paramagnetic particles to the second wall.

Preferably, in this particular embodiment, the second wall comprises further antibodies capable of immobilising the paramagnetic particles to which analyte has been bound.

It is not essential to the invention that the poles of the second magnet field is arranged in such a way as to form an angle (v₂) of less than 90° with the second wall. However, it is preferred that also the second magnet field is arranged according to the invention, i.e. in such a way that the second field generating means are arranged relative to the second wall so that the second pole axis to (a₂) form an angle (v₂) of less than 60° with the second wall. In one embodiment, the second field generating means are arranged relative to the second wall so that the angle (v₁) between the second pole axis and the second wall is between 0° and 60°.

In one embodiment, the second field generating means are arranged relative to the second wall so that the angle (v₁) between the second pole axis and the second wall is between 0° and 40°.

In one embodiment, the second field generating means are arranged relative to the second wall so that the angle (v₁) between the second pole axis and the second wall is between 0° and 20°.

In one embodiment, the second field generating means are arranged relative to the second wall so that the angle (v₁) between the second pole axis and the second wall is between 0° and 10°.

In one embodiment, the second field generating means are arranged relative to the first wall so that the angle (v₁) between the second pole axis and the first wall is 0°.

Preferably, the paramagnetic particles are coated with a receptor such as an antibody, which may bind to an analyte of interest. Preferably, the liquid comprises an analyte capable of binding to the paramagnetic particles. The liquid comprising an analyte capable of binding to the paramagnetic particles may be blood.

The problem faced by the inventors of the present invention is a problem especially associated with the manipulation of particles in very small liquid samples. In larger samples, problems with agglomeration of particles do not occur to the same extent as samples are readily shaken and thereby causing a homogeneous distribution of particles. In small volume samples on the contrary, shaking and otherwise mechanically manipulating samples is difficult or practically impossible, and may not lead to the desired disruption of agglomerates, as observed when using the method according to the invention.

Thus, in preferred embodiments of the invention, the small volume liquids are defined as liquids having a volume of 0-500 µl, particularly 0-400 µl, more particularly 0-300 µl, more particularly 0-200 µl, more particularly 0-150 µl, more particularly 0-100 µl, more particularly 0-50 µl, more particularly 0-30 µl.

Thus, in preferred embodiment, the chamber containing a plurality of paramagnetic particles is suited for samples having a volume of less than 200µl. A chamber is suited for samples having a volume of less than 200µl if substantially all of the plurality of paramagnetic particles are suspended in the sample when the sample is applied to the chamber. Thus, in a preferred embodiment of the invention, the sample to be analysed preferably has a volume of less than 200µl. In an even more preferred embodiment, the sample to be analysed has a volume of less than 150µl, even more preferred less than 100µl, even more preferred less than 90µl, such as less than 80µl, less than 70µl or even less than 60µl. In an even more preferred embodiment the sample to be analysed has a volume of less than 50µl, even more preferred less than 45µl, even more preferred less than 40µl.

In one embodiment, the chamber is a capillary channel. In a preferred embodiment, the chamber has a volume of less than 100µl. In an even more preferred embodiment, the chamber has a volume of less than 90µl, even more preferred less than 80µl, even more preferred less than 70µl, such as less than 60µl, less than 50µl or even less than 40µl. In an even more preferred embodiment, the chamber has a volume of less than 30µl, even more preferred less than 25µl, even more preferred less than 20µl, such as less than 15µl, less than 10µl or even less than 5 µl.

The method according to the invention is preferably performed in a system comprising a chamber device and a magnetic device. Thus, in one embodiment, the invention relates to a system for clustering and subsequently distributing a plurality of paramagnetic particles in a chamber, said system comprising a chamber device, and a magnetic device, said chamber device comprising a first wall and an opposite second wall, defining a chamber arranged for receiving and containing the plurality of paramagnetic particles, and said magnetic device comprising a) receiving means for receiving said chamber device and b) a first magnetic field generating means for generating a first magnetic field having first and second mutually spaced opposite poles, defining a first pole axis a₁) extending between the poles, the first field generating means being arranged relative to the first wall of a the chamber device, received in the receiving means, so that paramagnetic particles in the chamber are subjected to the field, and so that the first pole axis (a₁) forms an angle (v₁) of less than 60° with the first wall of the chamber device received in the receiving means, and further comprising means for changing the magnetic field.

By means for changing the magnetic field is meant e.g. means for moving the magnetic field in relation to the chamber device received in the receiving means, and/or means for removing the magnetic field, and/or means for reversing the polarity of the magnetic field.

The means for changing the magnetic field may comprise means for applying and removing the magnetic field. The means for changing the magnetic field may also comprise means for moving the first field generating means in a first moving direction having a movement component in the direction of the first pole axis. The means for changing the magnetic field may also comprise means for reversing the polarity of the magnetic field.

Removing the magnetic field may be accomplished by rapid movement of the means for moving the first field generating means in a first moving direction having a movement component in the direction of the first pole axis. Removal may also be accomplished by means of moving the field generating means away from the chamber in a vertical direction or by means for switching of the power in case of electromagnets.

In one embodiment, the system according to the invention comprises a magnetic device wherein the magnetic device comprises a second magnetic field generating means for generating a second magnetic field having first and second mutually spaced opposite poles and defining a second pole axis (a₂) extending between the poles, the second field generating means being arranged relative to the second wall of a the chamber device, received in the receiving means, so that paramagnetic particles in the chamber are subjected to the second field, so that the particles are attracted to the second wall.

Preferably, if two or more field generating means are used at least one must be arranged according to the invention. Accordingly, one embodiment of the invention relates to a system wherein the first field generating means is arranged relative to the first wall so that an angle (v₁) of less than 60° is formed between the first pole axis and the first wall of the chamber device received in the receiving means, and optionally wherein the second field generating means is arranged relative to the second wall so that an angle (v₂) of less than 60° is formed, between the second pole axis and the second wall of the chamber device received in the receiving means.

Preferably, if there are two or more field generating means, they are all arranged according to the invention. Accordingly, in one embodiment, the second field-generating means are arranged relative to the second wall so that the angle (v₁) between the second pole axis and the second wall is between 0° and 60°, such as between 0° and 40°, such as between 0° and 20°, such as between 0° and 10°, such as 0°.

Preferably, the chamber is a capillary channel containing the paramagnetic particles and having a first end connected to a sample inlet and a second end connected to a solution inlet.

In one embodiment of the invention, the inventive instrument may be described as a device or apparatus comprising a) a channel device comprising a longitudinal capillary channel containing a plurality of paramagnetic particles, and b) a magnetic device comprising receiving means for receiving the channel device and comprising magnetic field generating means for generating a magnetic field.

Further, the invention relates to a method for the detection of the presence or absence of a target analyte in a sample consisting of less than 200 µl liquid, comprising the steps of:
a) providing a liquid comprising an analyte and a plurality of paramagnetic particles capable of binding and immobilising the analyte and ;
b) subjecting the paramagnetic particles comprising the immobilised analyte to a first magnetic field by means of a first field generating means having first and second mutually spaced opposite poles and defining a first pole axis extending between the poles, the field generating means being arranged relative to the first wall so that the first pole axis (a₁) forms an angle (v₁) of less than 60° with the first wall;
c) moving the magnetic field in a first moving direction having a movement component in the direction of the first pole axis, or alternatively, reversing the polarity of the magnetic field, and
d) optionally, moving the paramagnetic particles to a separate chamber comprising a washing and/or detection liquid or, alternatively, replacing the residual liquid in the chamber with a washing and/or detection liquid,
e) removing the magnetic field, and
f) detecting the analyte

In step b), the particles are clustered allowing manipulations of the particles and/or the residual sample material e.g. by removing the residual sample material and replacing it with other solutions or buffers or e.g. by attracting the particles to a particular surface to which some of the particles containing certain bound species may be bound.
The means for detecting the analyte species may be selected among surface acoustic wave (SAW) detectors, spectrophotometers, fluorometers, CCD sensor chip(s), CCOS sensor chip(s), PMT detector(s), or any suitable light detector.

In a preferred embodiment, the chamber comprising the liquid comprising the plurality of paramagnetic particles is a capillary channel having a cross section area of less than 1 mm², preferably 0.1-0.7 mm². In one embodiment, the width and height of the capillary channel is about 1.0 mm and about 0.2 mm, respectively. The length of the capillary channel is 5-50 mm. In one embodiment, the length of the capillary channel is 5-20 mm. In one embodiment, the length of the capillary channel is 12-20 mm.

In a further embodiment the invention relates to the use of the inventive system for the quantitative detection of the presence or absence of a target analyte in a sample.

### Examples

### Example, CV-values when detecting brain natriuretic peptide (BNP) in human plasma:

### Principle of the assay

The BNP assay is a two-side sandwich immunoassay using direct chemiluminescence technology and constant amounts of two monoclonal antibodies. The first antibody (tracer) is a HRP-labelled monoclonal mouse anti-human BNP antibody specific to the ring structure of BNP (KY). The second antibody in the solid phase is a biotinylated monoclonal mouse anti-human antibody specific to the C-terminal portion of the BNP, which is coupled to streptavidin coated paramagnetic particles. A direct relationship exists between the amount of BNP present in the plasma sample and the amount of relative light units (RLUs) detected by the Photo Multiplier Tube (PMT, Hamamatsu).

### The chamber device and pre treatment of the capillary channel

18 chamber devices (se figure 1 and 2) were fabricated in black and the detection window (14) of the device in clear ABS (acryl nitrile butadiene styrene) polymer. The overall length, height and thickness of the chamber device were approximately 70, 35 and 2 mm respectively. The total length of the capillary channel (3, 5, 6) was approximately 40 mm, the width of the entire channel (3, 5, 6) was approximately 1.0 mm. The depth of the first part of the channel (3) was approximately 0.2 mm and the depth of all other parts (5, 6) of the channel was approximately 0.5 mm. The channel device was washed ultrasonically in a 50vol% water solution of 2-propanol and corona treated 25W/2s in order to increase the hydrophilicity prior to dispensing the paramagnetic particles.

After corona treatment, a solution of monoclonal mouse antibody (antibody against BNP) coated paramagnetic particles in a buffer (TBS: 0.05wt% Mouse IgG, 0.05wt% Bovine Gamma Gglobulin, 5 wt% sucrose, 5wt% BSA, 0.05vol% Tween, 1wt% Polyoxyetylene 10 tridecyl ether and 0.1wt% ProClin 300 (preservative)) was made and approximately 1µl of this solution was applied to the channel with a dispenser (Nanodrop ExtY) in such a way that only the first part (3) of the channel was covered with the solution. The solution was left to dry up completely for approximately 15 minutes. The device was then sealed with a pressure sensitive adhesive film (PSA).

### Conducting the assay

Human plasma with a BNP concentration of 20 pg/mL was added to a solution of monoclonal mouse BNP antibody "KY" covalently coupled to approximately one Horse Radish Peroxide molecule each. BNP and mouse antibody KY now specifically coupled in the solution. After approximately 1 minute of reaction time the human plasma solution was let in through the first end (1). The plasma now dissolved the sugar matrix in the chamber and thereby re-suspended the paramagnetic particles in the chamber.

The 18 chamber devices were divided in 2 groups of 9 each.

Either Magnet A (se figure 5) (group A) or Magnet B (se figure 5) (group B) was now put on top of the PSA and moved mechanically back and forth over the chamber (3).

In magnet A group the poles of the magnetic field are both placed directly perpendicular to the flow direction of the liquid in the chamber device. In magnet B group the poles of the magnetic field are arranged so that they are mutually spaced viewed in the longitudinal direction of the capillary channel. The first angle (v1) to the chamber was 0 Degrees (fig. 5A and 5B).

This motion of the magnet was continued for approximately 200 seconds. During this period of time the BNP molecule formed a sandwich between the antibody KY and the antibody specific couple to the paramagnetic particles.

A washing buffer (TBS: 0.05wt% BSA, 0.05vol% Tween, and 0.1wt% Kathon or 0.1wt% ProClin 300) was let in trough inlet (8, 25, 26) and filled the measuring area, the washing chamber and approximately 50% of the waste chambers. The magnet was then moved slowly from the chamber (3) to the washing chamber (5) in such a way that all the paramagnetic particles suspended were in close contact with the magnet as the washing chamber (5) was reached. The magnet was now moved back and forth between the ends (from (4a) to (6) and vice versa) of the washing chamber (5) in such a way that a minimum of paramagnetic particles was left in close contact with the magnet at the turning points for approximately 20s. The magnet was now moved very fast away from the chamber device and the paramagnetic particles fell via gravity to the bottom of the chamber. The washing cycle was repeated once.

The magnet was then moved from the capillary chamber (5) to the center of the measuring area (14) of the detection part of the chamber (6) in such a way that all the paramagnetic particles suspended were in close contact with the magnet as the center of the measuring area (14) was reached. A chemiluminescence substrate (Supersignal Elisa Femto Maximum Sensitivity Substrate from Pierce prod. 37074), which produces photons when brought in contact with HRP, was added in order to completely fill up the measuring area (6, 14), the washing chamber (5) and approximately 50% of the waste chambers (27). The magnet was then moved back and forth between the ends (the entire length of (6)) of the measuring area in such a way that a minimum of paramagnetic particles was left in close contact with the magnet at the turning points for approximately 8s. The magnet was now moved away very fast from the capillary chamber and the paramagnetic particles precipitate via gravity to the bottom of the chamber.

As soon as the magnet was moved away very fast from the measuring area, the PMT located under the measuring area was turned on and a measurement of the number of photons traveling through the window was performed for 120s.

The results of the two experiments are illustrated in table 1 below.

**Table 1. Measured Relative Light Units (RLU) after 40 seconds of measurement expressed as the Mean Value, Standard deviation and CV value of the RLU's.**

| | Magnet A | Magnet B |
|---|---|---|
| 1 | 279622 | 395732 |
| 2 | 308120 | 357968 |
| 3 | 215242 | 386774 |
| 4 | 110194 | 444728 |
| 5 | 171108 | 425300 |
| 6 | 171836 | 386988 |
| 7 | 179160 | 376776 |
| 8 | 134124 | 402616 |
| 9 | 355194 | 422506 |
| Mean Value | 213844 | 399932 |
| Std. | 78236 | 25435 |
| CV Value | 36,6% | 6,4% |

### Results and conclusion

The results show that the signal was about 1.9 times higher in experiment B compared to experiment A and the standard derivation was about 3.1 times lower. This resulted in a remarkable smaller CV value (standard deviation/mean) about 5.7 times lower in experiment B compared to experiment A. The characteristics of the curve were also different for the two experiments. In experiment A, the maximal RLU value was reached after about 7 s and remained at the maximal level until 15 s, resulting in a plateau at about 8 s. In experiment B, the maximal RLU value was reached after about 20 s and remained at the maximal level until 40 s, resulting in a plateau at about 20 s.

All the mentioned characteristics reflect the expected differences in the behaviour of paramagnetic particles in clusters (experiment A) versus the behaviour of paramagnetic particles in smaller clusters and as single particles. Thus, in the latter situation the shield of light signals is expected to be smaller resulting in higher signal, the clustering of paramagnetic particles is lowered and the sedimentation of the paramagnetic particles is slower resulting in a longer plateau phase for the maximal RLU value.

This experiment clearly demonstrates the significant improvement of signal strength and CV value when using a magnetic field which is moved with its field lines substantially perpendicular to the flow direction compared to the situation where the field lines are substantially parallel to the flow direction.

## Claims

1. Method for clustering and subsequently distributing a plurality of paramagnetic particles in a small volume liquid in a chamber, said chamber having a first wall and an opposite second wall, said method comprising the following steps;
a) providing a liquid comprising the plurality of paramagnetic particles;
b) subjecting the paramagnetic particles to a first magnetic field by means of a first field generating means having first and second mutually spaced opposite poles and defining a first pole axis extending between the poles, the field generating means being arranged relative to the first wall so that the first pole axis (a₁) forms an angle (v₁) of less than 60° with the first wall;
c) moving the magnetic field in a first moving direction having a movement component in the direction of the first pole axis, or, alternatively, changing the polarity of the magnetic field one or more times, and
d) removing the magnetic field.

2. Method according to claim 1, wherein steps a) to d) are repeated one or more times.

3. Method according to claim 1 or 2 comprising subsequent to step c) and before step d), a step c₁) of moving the magnetic field in a reverse moving direction having a movement component in the reverse direction of the first pole axis (a₁).

4. Method according to claim 3, wherein the steps c) and c₁) are repeated one or more times.

5. Method according to any of the preceding claims, wherein the first field generating means are arranged relative to the first wall so that the angle (v₁) between the first pole axis and the first wall is between 0° and 60°, particularly between 0° and 40°, particularly between 0° and 20°, particularly between 0° and 10°, and particularly 0°.

6. Method according to any of the preceding claims, wherein the first moving direction is essentially in the direction of the first pole axis a₁.

7. Method according to any of the preceding claims, wherein the reverse moving direction is essentially in the reverse direction of the first pole axis a₁.

8. Method according to any of the preceding claims comprising subsequent to step d), a step e) of subjecting the paramagnetic particles to a second magnetic field by means of a second field generating means having first and second mutually spaced opposite poles and defining a second pole axis a₂ extending between the poles, the second field generating means being arranged relative to the second wall so as to attract the paramagnetic particles to the second wall.

9. Method according to any of the preceding claims, wherein the small volume liquid comprises an analyte capable of binding to the paramagnetic particles and has a volume of 0-500 µl, particularly 0-400 µl, more particularly 0-300 µl, more particularly 0-200 µl, more particularly 0-150 µl, more particularly 0-100 µl, more particularly 0-50 µl, more particularly 0-30 µl.

10. System for clustering and subsequently distributing a plurality of paramagnetic particles in a chamber, said system comprising a chamber device, and a magnetic device, said chamber device comprising a first wall and an opposite second wall defining a chamber arranged for receiving and containing the plurality of paramagnetic particles, and said magnetic device comprising a) receiving means for receiving said chamber device and comprising a first magnetic field generating means for generating a first magnetic field having first and second mutually spaced opposite poles, and defining a first pole axis a₁ extending between the poles, the first field-generating means being arranged relative to the first wall of a the chamber device received in the receiving means so that paramagnetic particles in the chamber are subjected to the field and so that the first pole axis a₁ forms an angle (v₁) of less than 60° with the first wall of the chamber device received in the receiving means, and further comprising means for changing the magnetic field.

11. System according to claim 10, wherein the means for changing the magnetic field comprise means for applying and removing the magnetic field.

12. System according to claim 10 or 11, wherein the means for changing the magnetic field comprises means for moving the first field generating means in a first moving direction having a movement component in the direction of the first pole axis, or, alternatively, means for changing the polarity of the magnetic field one or more times.

13. System according to any of the preceding claims 10-12, wherein the magnetic device comprises a second magnetic field generating means for generating a second magnetic field having first and second mutually spaced opposite poles and defining a second pole axis (a₂) extending between the poles, the second field-generating means being arranged relative to the second wall of a the chamber device received in the receiving means, so that paramagnetic particles in the chamber are subjected to the second field, so that the particles are attracted to the second wall.

14. System according to any of the preceding claims 10-13, wherein the chamber is a capillary channel containing the paramagnetic particles and having a first end connected to a sample inlet and a second end connected to a solution inlet.

15. Method for the detection of the presence or absence of a target analyte in a sample consisting of less than 200 µl liquid, comprising the steps of;
a) providing a liquid comprising an analyte and a plurality of paramagnetic particles capable of binding and immobilising the analyte and ;
b) subjecting the paramagnetic particles comprising the immobilised analyte to a first magnetic field by means of a first field generating means having first and second mutually spaced opposite poles and defining a first pole axis extending between the poles, the field generating means being arranged relative to the first wall so that the first pole axis (a₁) forms an angle (v₁) of less than 60° with the first wall;
c) moving the magnetic field in a first moving direction having a movement component in the direction of the first pole axis, or alternatively, reversing the polarity of the magnetic field, and
d) optionally, moving the paramagnetic particles to a separate chamber comprising a washing and/or detection liquid or, alternatively, replacing the residual liquid in the chamber with a washing and/or detection liquid,
e) removing the magnetic field, and
f) detecting the analyte.
